# EUROPÄISCHE PATENTSCHRIFT

(11) **EP 1 178 105 B1**
(45) Veröffentlichungstag und Bekanntmachung des Hinweises auf die Patenterteilung: **25.10.2006**
(21) Anmeldenummer: 01117411.7
(22) Anmeldetag: 19.07.2001
(51) Int. Cl.: C11B 9/00, C07C 43/18, C07C 43/188

(54) **Isolongifolenylether, ihre Herstellung und ihre Verwendung**
Isolongifolenyl ethers, their preparation and their use
Ethers d'isolongifolenyle, leur préparation et leur usage

(30) Priorität: 03.08.2000 DE 10038544
(43) Veröffentlichungstag der Anmeldung: 06.02.2002
(73) Patentinhaber: Symrise GmbH & Co. KG, 37603 Holzminden (DE)
(72) Erfinder: Pickenhagen, Wilhelm, Dr., 37671 Höxter (DE); Schatkowski, Dietmar, 37627 Stadtoldendorf (DE)
(74) Vertreter: Eisenführ, Speiser & Partner

(56) Entgegenhaltungen:
- EP-A- 0 669 308
- GB-A- 1 256 535
- US-A- 4 439 354
- S. ARCTANDER: "PERFUME AND FLAVOR CHEMICALS (AROMA CHEMICALS). " 1969 , S. ARCTANDER , MONTCLAIR ; US XP002256490 * Beispiele 598,599 *
- BOMBARDA I ET AL: "STRUCTURE ELUCIDATION OF OXIDATION-REDUCTION PRODUCTS OF ISOLONGIFOLENE" JOURNAL OF AGRICULTURAL AND FOOD CHEMISTRY, AMERICAN CHEMICAL SOCIETY. WASHINGTON, US, Bd. 42, Nr. 1, 1994, Seiten 138-142, XP000420727 ISSN: 0021-8561

## Beschreibung

Industriell hergestellte Parfümöle bestehen heutzutage größtenteils aus synthetischen Riechstoffen.

Die traditionelle Verwendung von etherischen Ölen und Extrakten pflanzlichen und tierischen Ursprungs spielt zwar auf dem Gebiet der alkoholischen (Fein-)Parfümerie immer noch eine wichtige Rolle, die Parfümierung von Waschmitteln, Seifen, Haushaltsreinigern und ähnlichen technischen Produkten erfordert aber den Einsatz von Riechstoffen, die den technischen Anforderungen hinsichtlich Stabilität und Substantivität genügen. Um diesen speziellen Anforderungen zu entsprechen, werden Parfüms zur Verwendung in technischen Konsumgütern überwiegend aus synthetischen Riechstoffen formuliert. Weil diese Parfüms aber in großen Mengen benötigt werden, haben alle bedeutenden Parfümhäuser und Riechstoff-Hersteller von Aromastoffen in den letzten Jahrzehnten ihre Forschungsaktivitäten auf die Herstellung neuer synthetischer Riechstoffe gerichtet.

Zwischenzeitlich haben synthetisch hergestellte Riechstoffe, die in der Vergangenheit aufgrund ihres vergleichsweise niedrigen Preises und ihrer hohen Stabilität im wesentlichen zur technischen Anwendung hergestellt wurden, auch zunehmend Eingang in die Feinparfümerie gefunden.

Inzwischen besteht ein ständiger Bedarf an Riechstoffen mit verbesserten Eigenschaften wie Geruchsqualität, Stabilität, Hautverträglichkeit und Umweltverträglichkeit. Im Sinne einer ausgeglichenen Öko-Bilanz werden dabei Produkte angestrebt, die auf nachwachsenden Rohstoffen basieren.

Ein in relativ großen Mengen verfügbarer Rohstoff natürlichen Ursprungs ist das Sesquiterpen Longifolen (**1**), das als Hauptkomponente in indischem Terpentinöl und als Nebenkomponente in zahlreichen anderen Terpentinölsorten sowie weiteren etherischen Ölen vorkommt. Bereits vor mehr als 2 Jahrzehnten wurde eine Reihe von Folgeprodukten aus Longifolen hergestellt und deren Riechstoffeigenschaften beschrieben. G. Ohloff berichtet in seinem Buch "Riechstoffe und Geruchssinn" [Springer-Verlag, 1990, ISBN-Nr. 3-540-52560-2, Seiten 87 - 88] zusammenfassend, das von (+)-Longifolen (**1**) mindestens 4 kommerzielle Riechstoffe abstammen und von dem hieraus durch Isomerisierung zugänglichen Isolongifolen (**2**) 13 Riechstoffderivate kommerziell genutzt werden. Zu einzelnen dieser Derivate gibt Ohloff den charakteristischen Geruchstyp als holzig (Holzgeruch) an; andere Geruchsnoten werden nicht erwähnt.

Über Chemie und Geruchseigenschaften der kommerziell wichtigen Derivate des Isolongifolens (**2**) berichten G. Färber und H. Tan [G. Färber, Parfümerie + Kosmetik, 68, 18 (1987), H. Tan, Parfümerie + Kosmetik, 67, 564 (1986)]. Dabei läßt sich insbesondere der letztgenannten Veröffentlichung entnehmen, daß die als Riechstoff bekannten Isolongifolen-Derivate aufgrund ihres holzigen Charakters anstelle der bekannten Holz-Duftnoten eingesetzt werden können.

In unserer Veröffentlichung EP-A 0 543 470 sind cyclische Isolongifolanon-Ketale der allgemeinen Formel B 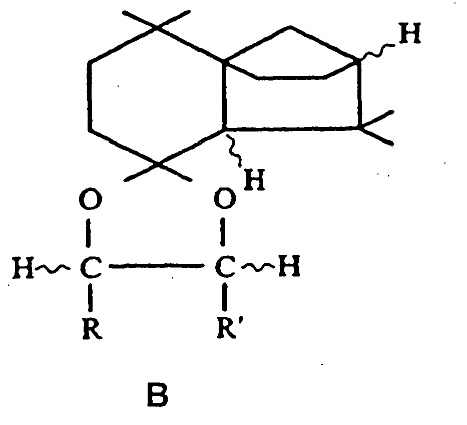 (in der geschlängelte Linien α- und β-Konfiguration sowie R- und R' Wasserstoff- bzw. Methyl- oder Ethylreste bedeuten) beschrieben. Diese Ketale sind wertvolle Riechstoffe, die stark holzige Geruchseigenschaften mit blumig-frischen Effekten und samtigen Moos-/Ambra-Charakter besitzen.

In unserer Veröffentlichung EP-A 0 669 308 sind Isolongifolanol-Derivate der allgemeinen Formel C 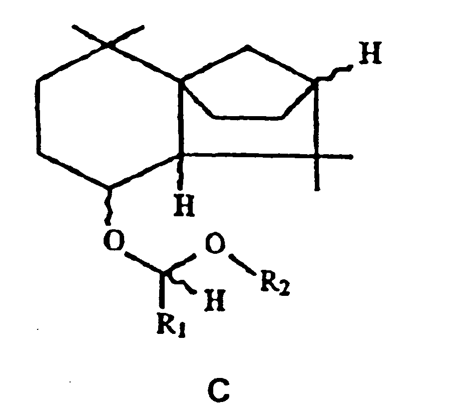 (in der geschlängelte Linien α- und β-Konfiguration sowie R1 Wasserstoff oder Methylreste und R2 Methyl- oder Ethylreste bedeuten) beschrieben. Diese Derivate besitzen ebenfalls holzige olfaktorische Eigenschaften.

In der GB-A 1 505 821 sind Isolongifolanol und dessen niedrige Ester (Acylgruppen mit bis zu 6 C-Atomen) als Riechstoffe beschrieben, wobei auf die α/β-Isomerie der Hydroxyl- bzw. Carboxylfunktion eingegangen wird. Die genannten Riechstoffe besitzen starke holzige, an Cedrylacetat und Vetiverylacetat erinnernde Geruchseigenschaften und sind besonders gut für Parfümkompositionen mit blumigem oder Citrus-holzigem Charakter geeignet.

Die GB-A 1 256 535 beschreibt die kontrollierte Oxidation von Isolongifolen (**2**) zu Isolongifolenon (**3**) und dessen anschließende Reduktion zu Isolongifolenol (**4**). Die anschließende Veresterung (Acylgruppen mit bis zu 5 C-Atomen) des nahezu geruchlosen Isolongifolenol (**4**) führt wiederum zu Riechstoffen (Isolongifolenyl-Estern) mit holzigem Geruchsprofil.

Entsprechend dem soeben genannten Stand der Technik gilt das Gebiet der Longifolen- und Isolongifolen-Derivate als besonders gut untersucht. Neben einer begrenzten Zahl von Longifolen- und Isolongifolen-Derivaten mit wertvollen Riechstoffeigenschaften (vgl. insbesondere den vorstehend genannten Stand der Technik) sind eine unübersichtlich große Zahl von Derivaten der vorgenannten Sesquiterpene in der Literatur beschrieben oder von uns synthetisiert und untersucht worden, die kaum oder keinen wesentlichen olfaktorischen Wert besitzen.

Wenngleich einzelne der bekannten Isolongifolen-Derivate hervorragende olfaktorische Eigenschaften besitzen und in industriellem Maßstab sowohl preiswert als auch umweltverträglich herstellbar sind, wurde es doch mit Blick auf die Gruppe der Isolongifolen-Derivate als Ganze zunehmend als nachteilig und begrenzend empfunden, daß bei Isolongifolenol-Derivaten mit einer nennenswerten olfaktorischen Aktivität regelmäßig ein holziger Aspekt im Vordergrund steht, der das Einsatz-Spektrum der Derivate einschränkt.

Es war daher die primäre Aufgabe der vorliegenden Erfindung, einen Riechstoff, vorzugsweise ein Isolongifolen-Derivat oder eine Gruppe von Isolongifolen-Derivaten und ein Verfahren zu dessen Herstellung anzugeben, wobei im Vergleich mit den zuvor genannten olfaktorisch interessanten Isolongifolen-Derivaten zumindest nicht nur die holzigen, sondern auch andere Geruchs-Aspekte im Vordergrund stehen sollten. Vorteilhafterweise sollten dabei die nicht-holzigen Aspekte dominieren.

Damit im Zusammenhang stand die weitere Aufgabe, eine Parfümkomposition anzugeben, deren olfaktorischen Eigenschaften durch einen Riechstoff, vorzugsweise ein Isolongifolen-Derivat oder eine Mischung von Isolongifolen-Derivaten mitbestimmt werden, bei dem bzw. denen zumindest nicht nur holzige sondern auch andere Geruchs-Aspekte im Vordergrund stehen.

Weitere der vorliegenden Erfindung zugrundeliegende Aufgaben lassen sich wie folgt formulieren:

Das Isolongifolen-Derivat oder die Mischung von Isolongifolen-Derivaten sollte möglichst zu einem lang anhaftenden Nachgeruch und einer guten Fixierung einer Parfümkomposition beitragen können.

Das Isolongifolen-Derivat oder die Mischung von Isolongifolen-Derivaten sollte möglichst in einer Parfümkomposition zu einer Abrundung und Harmonisierung der Hauptnote (des "Bouquets") beitragen können.

Eine weitere Aufgabe der vorliegenden Erfindung bestand schließlich darin, ein Verfahren anzugeben, mit dem einem Material (einer Substanz) ein (nicht primär holziger) Geruch hinzugefügt oder dessen (Eigen-)Geruch verstärkt wird, indem zu dem Material (der Substanz) ein Isolongifolen-Derivat oder eine Mischung von Isolongifolen-Derivaten hinzugegeben wird.

Ein besonders großes Interesse bestand dabei an Riechstoffen mit Moschus-Charakter. Die bislang als Riechstoffe eingesetzten Isolongifolen-Derivate besitzen regelmäßig keinen derartigen Moschus-Charakter. Zur Herstellung von Parfümölen mit Moschusnote ist es daher erforderlich, einen der bekannten Moschusriechstoffe mit dem jeweiligen Isolongifolen-Derivat zu mischen.

So wird beispielsweise gemäß der bereits erwähnten GB-A 1 256 535 u.a. empfohlen, die dort beschriebenen Isolongifolenol-Ester mit einer Moschus-Verbindung wie Moschus-Keton oder Ethylenbrassylat zu vermischen.

In der ebenfalls bereits erwähnten GB-A 1 505 821 gehören ebenfalls Moschus-Verbindungen zu den bevorzugten Verbindungen zum Vermischen mit den offenbarten Isolongifolen-Derivaten.

Auch hinsichtlich der in den genannten eigenen Veröffentlichungen offenbarten Verbindungen ist die Situation jeweils ähnlich.

Erfindungsgemäß wird die gestellte Aufgabe gelöst durch Angabe und Verwendung der neuen Verbindung der allgemeinen Formel **A** 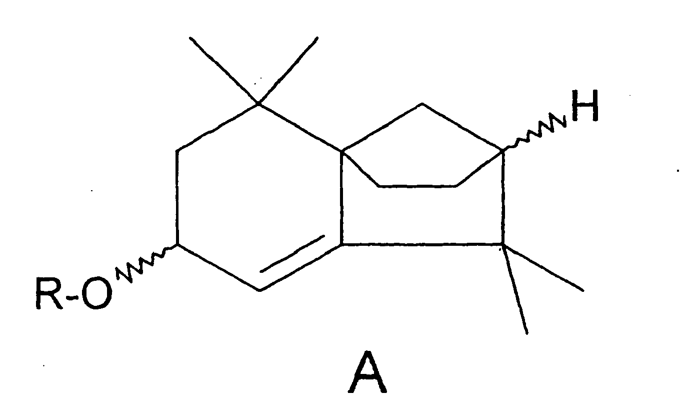 (in der geschlängelte Linien α- und β-Konfiguration sowie R = Methyl-, Ethyl-, Propyl-, iso-Propyl-, Butyl-, sec-Butyl-, iso-Butyl-, tertiär-Butyl-Reste bedeuten).

Die unter diese allgemeine Formel fallenden Einzelverbindungen und deren Mischungen (d.h. Enantiomerengemische und/oder Mischungen von reinen oder gemischten Enantiomeren mit unterschiedlichen Resten R) verfügen über ganz eigenständige geruchliche Eigenschaften und unterscheiden sich hierbei auch deutlich von den vorstehend diskutierten, bekannten Riechstoffen aus der Familie der lsolongifolenol-Derivate. So verfügen die neuen unter die allgemeine Formel **A** fallenden Einzelverbindungen interessanterweise nur über sehr schwache holzige Geruchseigenschaften. Stattdessen zeichnen Sie sich durch einen starken, weichen, samtigen Moschus/Ambra-Geruch aus; in einigen Fällen ist im Nachgeruch auch ein Patchouli-Aspekt festzustellen. Die vorstehende Geruchscharakteristik gilt insbesondere für (Einzel-)Verbindungen der allgemeinen Formel **A** mit R = Methyl, Ethyl-, Propyl-, und iso-Propyl (jeweils reines Enantiomer oder Enantiomererngemisch). Für Mischungen der genannten Einzelverbindungen mit unterschiedlichem Alkylrest in der Etherfunktion gilt entsprechendes.

In Parfümkompositionen mit blumigem Akzent verstärken die unter die allgemeine Formel **A** fallenden Einzelverbindungen und deren Mischungen außerdem synergistisch den blumigen Effekt und frischen diesen auf, insbesondere in der Kopfnote.

Die unter die allgemeine Formel **A** fallenden erfindungsgemäßen Einzelverbindungen (reine Enantiomere oder Enantiomerengemische) sind besonders lang haftend und fixierend.

Cyclische Isolongifolenylether der allgemeinen Formel **A** mit R = Me, Et, Pr oder Iso-Pr, bei denen die Konfiguration der Etherfunktion α oder β ist oder der cyclische Isolongifolenylether eine Mischung von Enantiomeren umfaßt, die sich in der Konfiguration der Etherfunktion unterscheiden, sind dabei allerdings im Vergleich mit den Ethern (reines Enantiomer oder Enantiomerenmischung) der allgemeinen Formel A mit R = Butyl-, sec-Butyl-, iso-Butyl-,oder tertiär-Butyl bevorzugt.

Unter ihnen sind wiederum die isomeren Ether der allgemeinen Formel A mit R = Methyl (reines Enantiomer oder Enantiomerenmischung) aufgrund
(a) ihrer besonderen, insbesondere den Nachgeruch einer Parfümkomposition beeinflussenden Geruchscharakteristik (mit Moschus-Aspekten),
(b) ihres hohen Fixierungsvermögens sowie ihrer guten Anhaftung auf üblichen Substraten,
(c) ihrer Einsetzbarkeit zur Verstärkung insbesondere blumiger Kopfnoten sowie
(d) ihrer Einsetzbarkeit zur Abrundung und Harmonisierung bestimmter Hauptnoten für den Einsatz in der Parfümerie besonders bevorzugt.

Die isomeren Ether der allgemeinen Formel A mit R = Ethyl, Propyl- oder iso-Propyl (reines Enantiomer oder Enantiomerenmischung) besitzen ebenfalls eine überraschend ausgeprägte, den Nachgeruch einer Parfümkomposition beeinflussende Geruchscharakteristik, sind aber insgesamt nicht ganz so geruchsintensiv wie die Isomeren der allgemeinen Formel A mit R = Methyl. Sie sind daher insbesondere zur Abrundung und Harmonisierung bestimmter Hauptnoten vorgesehen.

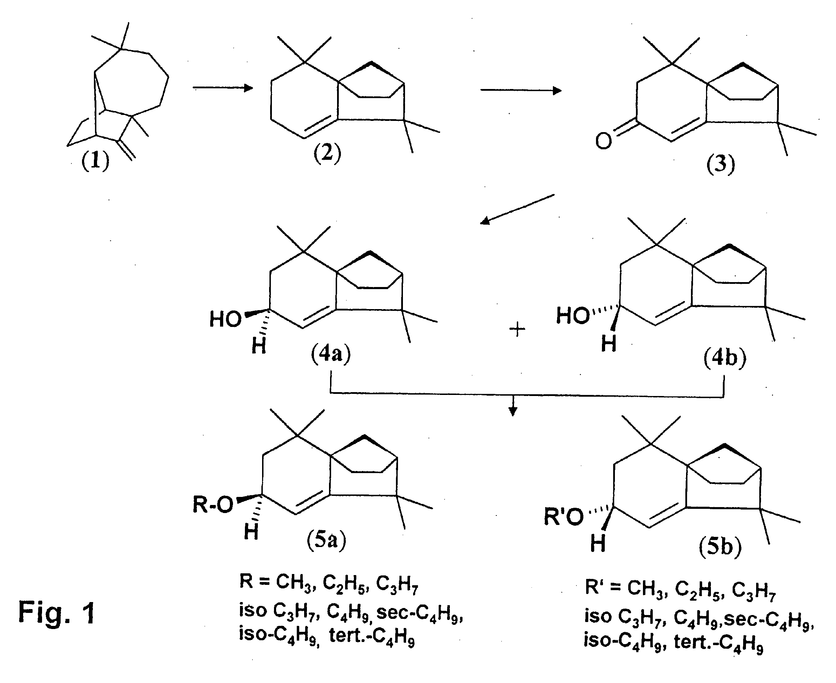

Zur Herstellung der unter die allgemeine Formel A fallenden Methylether wurde gemäß dem Verfahrensschema in Fig. 1 (+)-Longifolen (**1**) in bekannter Weise durch Behandeln mit einem Gemisch aus Essigsäure und Schwefelsäure (U.R. Nayak S. Dev, Tetrahedron **8**, 42-48 (1960)] oder mit Bortrifluorid-Etherat (R.E. Beyler, G. Ourisson, J. Org. Chem., 30, 2838-2839 (1965)] zu Isolongifolen (2) isomerisiert. Eine anschließende kontrollierte
(a) Oxidation von Isolongifolen (**2**) mit Natriumbichromat in Eisessig [DE 1804711] **oder**
(b) Luftoxidation von Isolongifolen (**2**) [Organikum **16**, 169-170 (1986)] (G. Färber "Riechstoffe aus Isolongifolen", Parfümerie + Kosmetik, **68**, 18 (1987)]
führte zum Isolongifolenon (**3**).

Isolongifolenon (**3**) wurde dann in an sich bekannter Weise zu einem Gemisch der epimeren Alkohole **4a** und **4b** reduziert, wobei das Massenverhältnis **4a : 4b** von den gewählten Reaktionsbedingungen abhing. Die chiralen Alkohole **4a/b** wurden als Gemisch oder in reiner Form in an sich bekannter Weise in die epimeren Methylether **5a** und **5b** überführt.

Die Herstellung der epimeren Ethyl-, Propyl-, iso-Propyl-, Butyl-, sec-Butyl-, iso-Butyl- und tertiär-Butyl-Ether (Verbindungen **6a/b - 12a/b**) erfolgte auf analoge Weise (vgl. die Herstellungsvorschriften weiter unten).

Die stereochemischen Verhältnisse bleiben bei der Umsetzung des Isolongifolenol-Epimerengemischs **4a/b** oder der reinen Epimerne **4a** oder **4b** zu den neuen Ethern der allgemeinen Formel **A** im wesentlichen unverändert, so daß auch die neuen Ether der allgemeinen Formel **A** abhängig von der stereochemischen Zusammensetzung des Isolongifolenol als Epimerengemische oder reine Epimere vorlagen. Mit Blick auf die Isolongifolenol-Epimerengemische **4a/b** ist noch zu beachten, daß in Abhängigkeit von den gewählten Reaktionsbedingungen bei der Reduktion des Isolongifolenon **(3)** die epimeren Alkohole **4a** und **4b** in unterschiedlichen Mengen anfallen können, so daß nach Veretherung die epimeren Methylether **5a** und **5b** ebenfalls in unterschiedlichen Mengenverhältnissen vorliegen können.

Entsprechend den vorstehenden Ausführungen wurden ausgehend von (+)-Longifolen **(1)** über die chiralen Isolongifolenole **4a/b** die enstprechenden ebenfalls chiralen Ether der allgemeinen Formel A erhalten. **(+)**-Longifolen **(1)** ist aufgrund seiner preiswerten Verfügbarkeit derzeit und dürfte auch zukünftig in der industriellen Praxis die bevorzugte Ausgangsverbindung zur Herstellung von Isolongifolenol-Derivaten sein. Bei Verwendung dieser Ausgangsverbindung liegt die Stereochemie der entsprechenden erfindungsgemäßen lsolongifolenyl-Ether mit Ausnahme des die Etherfunktion tragenden Kohlenstoffs fest. Der Wasserstoff am Brückenkopf der beiden Fünfringe ist α-konfiguriert.

Ausgehend vom epimeren, in Pinus ponderosa enthaltenen **(-)**-Longifolen wurden auf analoge Weise auch die entsprechenden, stereochemisch von den Verbindungen **5a/b-12a/b** verschiedenen Isolongifolenyl-Ether synthetisiert. Diese unterscheiden sich in ihren Eigenschaften nicht signifikant von den Ethern, die ausgehend von (+)-Longifolen **(1)** erhalten wurden. In Fig. 1 und dem nachfolgenden Text wird die Erfindung daher der Übersichtlichkeit halber nur anhand der auf (+)-Longifolen **(1)** basierenden Verbindungen erläutert. Für die ausgehend von (-)-Longifolen erhältlichen Verbindungen gilt jeweils entsprechendes.

In einem Verfahren zur Herstellung eines der erfindungsgemäßen cyclischen Isolongifolenylether wird also Isolongifolenol in enantiomerenreiner Form oder als Enantiomerengemisch entweder unter säurekatalysierten Bedingungen mit einem aliphatischen Alkohol (Alkylhydroxid) oder unter basenkatalysierten Bedingungen mit einem Alkylhalogenid oder Alkylsulfat in einem aprotischen Lösungsmittel umgesetzt, wobei die jeweilige Alkylfunktion dem Rest R des cyclischen Isolongifolenylethers der allgemeinen Formel **A** entspricht.

Die neuen Ether der Formel **A** besitzen jeweils in reiner Form oder als Stereoisomerengemische originelle Riechstoffeigenschaften und können in reiner Form oder als Isomerengemische vorteilhaft als Riechstoffe bzw. Bestandteil von Parfümölen eingesetzt werden.

### Beispiel 1

### Herstellung von Isolongifolen (2)

Zu einer auf 60 °C erhitzten Lösung aus 360 g Toluol und 40 g (0.28 mol) BF₃-Etherat wurden innerhalb von 60 Minuten 816 g (3.16 mol) (+)-Longifolen (**1**) (80 %, ex Terpentinöl indisch [α]_{D} = + 39,6 °) zugetropft, danach auf Raumtemperatur herunter gekühlt und mit Sodalösung und Wasser neutral gewaschen. Nach Trocknung über Na₂SO₄ wurde das Lösungsmittel unter vermindertem Druck abdestilliert. Es verblieben 800 g (74 % nach GC) Rohprodukt.

Gaschromatogramm (Shimadzu GC 14A), DB1,30 m, 100 - 240 °C, 10 °C/min
Rₜ = 5.16 Min., **2**(74%)

### Beispiel 2

### Herstellung von Isolongifolenon (3)

In einem Dreihalskolben mit Rückflußkühler und Tropftrichter wurden 800 g (2.90 mol) Isolongifolen (**2**) (74 %ig nach GC) aus Beispiel 1 und 1 I Ether vorgelegt. Danach wurde über einen Zeitraum von 1,5 - 2 Std. bei einer Temperatur von 15 - 20 °C eine Lösung aus 1,5 kg Wasser, 300 g H₂SO₄ 98 %ig und 500 g (1.69 mol) K₂Cr₂O₇ zugetropft, 20 Std. bei 20°C nachgerührt und aufgearbeitet. Die abgetrennte organische Phase wurde mit Sodalösung und Wasser neutral gewaschen, über Na₂SO₄ getrocknet und das Lösungmsittel unter vermindertem Druck abdestilliert. Es verblieben 680 g Rohprodukt.

### GC (Bedingungen siehe Beispiel 1)

3 Rₜ = 12.2 Min. (48%)

Destillation über eine 40 cm Metallfüllkörperkolonne ergab 286 g Kp₅ₘₘ 105 - 108 °C, Smp. 39 - 40 °C

GC (Bedingungen siehe Beispiel 1): **3** (96,8 %).

GC/MS: HP 5985 A DBWAX, 60 N, 60 m, 60 - 240 °C, 4 °C/min.

**3** Rₜ = 40.7 Min.
MS (70 eV): m/e (%) = 218 (60 M⁺), 203 (15), 189 (10), 175 (100), 162(73), 147 (72), 133 (30), 119 (33), 91 (36), 41 (25).
¹³C-NMR (CDCl3), Varian VXR 300): δ [ppm]: 24.56, 25.38, 25.76, 26.98 (CH3), 24.32, 27.83, 36.68, 49.88 (CH2), 46.49, 116.81 (CH), 34.41, 44.00, 58.59, 183.78, 199.98 (C)

### Beispiel 3

### Herstellung eines Gemisches von Isolongifolenol 4a/4b [2:3]

In einem Dreihalskolben mit Rückflußkühler, Tropftrichter und Thermometer wurden 100 g (0.44 mol) Isolongifolenon (**3**) (96.8 % nach GC) aus Beispiel 2 und 100 g Methanol vorgelegt. Danach tropft man über einen Zeitraum von1 Std. bei 40 - 50 °C eine Lösung aus 150 g Wasser, 1 g NaOH und 5 g (0.13 mol) NaBH₄ zu, rührt 6 Std. bei 40 - 50 °C nach, kühlt auf Rarumtemperatur herunter und arbeitet auf. Es wurde mit 200 g Wasser und 100 g Ether versetzt, die organische Phase abgetrennt, die Wässer mit 100 g Ether extrahiert, die vereinigten organischen Phasen neutral gewaschen und über Na₂SO₄ getrocknet. Anschließend wurde das Lösungsmittel unter vermindertem Druck abdestilliert. Es verblieben 98 g Rohprodukt.

### GC (Bedingungen siehe Beispiel 1): 4a (30.9 %), 4b (59.8 %)

Anschließende Feindestillation über eine 15 cm Vigreux-Kolonne ergab 88 g Kp₂ₘₘ 110-112 °C.

GC (Bedingungen siehe Beispiel 1): **4a** (32.4 %), **4b** (65.3 %)

GC/MS: Bedingungen siehe Beispiel 2

**4a** Rₜ = 38.86 Min.
MS (70 eV): m/e (%) = 220 (11, M⁺), 205 (11), 191 (3), 177 (18), 159 (9), 137 (100), 121 (30), 107 (15), 91 (18), 41 (22).

**4b** Rₜ = 40.14 Min.
MS (70 eV): m/e (%) = 220 (13, M⁺), 205 (12), 191 (3), 177 (19), 159 (9), 137 (100), 121 (29), 107 (16), 91 (18), 43 (23)

### Beispiel 4

### Gewinnung von Isolongifolenol 4b

Durch Umkristallisieren von 10 g des aus Beispiel 3 erhaltenen Isolongifolenols **4a** (32.4 %), **4b** (65.3 %) aus 10 g Hexanfraktion 63/80 wurden 5,8 g Isolongifolenol (**4b**) als farblose Kristalle erhalten, Smp 89 - 90 °C).

### GC (Bedingungen siehe Beispiel 1): 4b (98.1 %)

### GC/MS (Bedingungen siehe Beispiel 2)

**4b** Rₜ = 40.16 Min.
MS (70 eV): m/e (%): 220 (14, M⁺), 205 (13), 191 (4), 177 (23), 159 (11), 137 (100), 121 (31), 107 (17), 91 (19), 43 (23).

¹³C-NMR (CDCl₃), (Varian VXR 300): δ [ppm]: 24.84, 25.29, 26.64, 28.84 (CH3), 24.72, 28.90, 36.56, 44.26 (CH2), 46.81, 67.61, 11.46 (CH), 33.24, 41.94, 56.54, 159.32 (C).

### Beispiel 5

### Herstellung eines Gemisches von Isolongifolenol 4a/4b [1:10]

In einem Dreihalskolben mit Tropftrichter, Vigreux-Kolonne mit aufgesetztem Kolonnenkopf und Thermometer wurden 200 ml 1 molare Aluminiumisopropylat-Lösung vorgelegt und zum Sieden erhitzt. Danach wurde innerhalb von 3 Std. eine Lösung bestehend aus 44 g (0.195 mol) Isolongifolenon (**3**) (96.8 %ig aus Beispiel 2) gelöst in 200 ml Isopropanol zugetropft. Gleichzeitig wurde während des Zutropfens am Kolonnenkopf 150 ml Lösemittelgemisch abdestilliert. Anschließend wurde auf Raumtemperatur heruntergekühlt, das Reaktionsgemisch mit 200 g Wasser und 100 g Ether versetzt, die organische Phase abgetrennt, die Wässer nochmals mit 100 g Ether extrahiert und die vereinigten organischen Phasen 1 x mit 100 g 10 %iger HCI und danach mit Sodalösung und Wasser neutralisiert. Anschließend wurde über Na₂SO₄ getrocknet und das Lösungsmittel unter vermindertem Druck abdestilliert. Es verblieben 42 g Rohprodukt.

GC (Bedingungen siehe Beispiel 1): **4a** (8.9%), **4b** (82.3 %)

### Beispiel 6

### Herstellung eines Gemisches von Isolongifolenol 4a/4b [95:5]

In einem Dreihalskolben mit Rückflußkühler, Thermometer und Tropftrichter wurden 10 g (0.044 mol) Isolongifolenon (**3**) (96.8 % nach GC) aus Beispiel 2 und 50 ml THF wasserfrei unter Stickstoffzufuhr vorgelegt, auf 0 - 5 °C heruntergekühlt und bei dieser Temperatur mit 50 ml (0.05 mol) Lithium-tri-sec.Butylborhydrid (L-Selektride, Fa. Aldrich) versetzt. Anschließend wurden 4 Std. bei 0 - 5 °C nachgerührt, auf Eis ausgegossen und aufgearbeitet. Die organische Phase wurde abgetrennt, die Wässer mit 50 ml Ether extrahiert, die vereinigten organischen Phasen über Na₂SO₄ getrocknet und das Lösungsmittel unter vermindertem Druck abdestilliert. Es verblieben 9.8 g Rohprodukt.

GC (Bedingungen siehe Beispiel 1): **4a** (86.8 %), **4b** (4.2 %)

### Beispiel 7

### Gewinnung von Isolongifolenol 4a

2 g des aus Beispiel 6 gewonnenen rohen Isolongifolenol (**4a/4b**) wurden durch präparative Chromatographie gereinigt.

Chromatographiebedingungen: 200 g Kieselgel 60, Korngröße 0.04 - 0.063 (Fa. Merck, Art.-Nr. 9385)
Laufmittel Benzin/Essigester 9:1,
Einwaage 2 g
Ausbeute: 1,21 g

### GC (Bedingungen siehe Beispiel 1): 4a (99,1 %)

### GC/MS (Bedingungen siehe Beispiel 2)

**4a** Rₜ = 38.86 Min.
MS (70 eV): m/e (%) = 220 (11, M⁺), 205 (11), 191 (3), 177 (18), 159 (9), 137 (100), 121 (30), 107 (15), 91 (18), 41 (22).
¹³C-NMR (CDCl₃, Varian VXR-300): δ [ppm]: 25.51, 26.60, 26.90, 28.23 (CH3), 24.64, 28.29, 36.97, 43.17 (CH2), 46.49, 65.97, 113.21 (CH), 30.68, 42.32, 56.75, 159.76 (C).

### Beispiel 8

### Herstellung eines Gemisches von Isolongifolenylmethylether 5a/5b [2:3]

In einem Dreihalskolben mit Tropftrichter, Thermometer und Rückflußkühler wurden 20 g (0.089 mol) Isolongifolenol (**4a/b**) (97,7 % nach GC) aus Beispiel 3 und 100 ml Methyl-tert.-Butylether vorgelegt. Anschließend wurden portionsweise 4 g (0.1 mol) NaH 60 %ig (in Paraffin) zugesetzt, zum Sieden erhitzt und 2 h unter Rückfluß gerührt. Danach wurde auf 70 - 80 °C heruntergekühlt und über einen Zeitraum von 30 Min. 14,2 g (0.1 mol) Methyljodid zugetropft. Es wurde insgesamt 6 Std. unter Rückfluß gerührt, auf Raumtemperatur heruntergekühlt, auf Wasser ausgegossen und die organische Phase abgetrennt. Die Wässer wurden einmal mit 50 ml Ether extrahiert, die vereinigten organischen Phasen mit Wasser neutral gewaschen, über Na₂SO₄ getrocknet und das Lösungsmittel unter vermindertem Druck abdestilliert. Es verblieben 21,4 g Rohprodukt.

### GC (Bedingungen siehe Beispiel 1): 5a (20.8 %), 5b (53,4 %)

Anschließende Feindestillation an einer Drehbandkolonne ergab 11,8 g Kp₂ₘₘ 139 - 140 °C
D 20/4 = 0,9710
n 20/D = 1,4983

### GC (Bedingungen siehe Beispiel 1): 5a (37.6 %), 5b (59.1 %)

### GC/MS: Bedingungen siehe Beispiel 2

**5a** Rt = 28.59 Min.
MS (70 eV): m/e (%): 234 (18, M⁺), 219 (20), 191 (10), 159 (11), 151 (100), 135 (21), 119 (10), 105 (11), 91 (13), 41 (10).

**5b** Rt = 29.09 Min.
MS (70 eV): m/e (%): 234 (20, M⁺), 219 (18), 191 (9), 159 (10), 151 (100), 135 (23), 119 (10), 105 (12), 91 (14), 41 (10).

### Beispiel 9

### Gewinnung von Isolongifolenylmethylether 5b

2 g des aus Beispiel 8 erhaltenen Destillats **5a** (37.6 %), **5b** (59.1 %) wurden durch präparative Chromatographie gereinigt.

Chromatographiebedingungen: 300 g Kieselgel 60, Korngröße 0.04 - 0.063 (Fa. Merck, Art.-Nr. 9385)
Laufmittel: Benzin/Essigester = 98/2
Einwaage: 2 g
Ausbeute: 651 mg

### GC (Bedingungen siehe Beispiel 1): 5b (97.8 %)

### GC/MS: Bedingungen siehe Beispiel 2

5b Rₜ = 29.09 Min.
MS (70 eV): m/e (%): 234 (21, M⁺), 219 (19), 191 (9), 159 (11), 151 (100), 135 (23), 119 (10), 105 (12), 91 (19), 41 (10)
¹³C-NMR (CDCl₃, Varian VRX-300): δ [ppm]: 24.96, 25.27, 26.77, 28.85, 55.38 (CH₃), 24.70, 28.74, 36.57, 39.86 (CH₂), 46.83, 76.17, 111.82 (CH), 32.92, 42.01, 56.60, 159.39 (C).

### Beispiel 10

### Herstellung eines Gemisches von Isolongifolenylmethylether 5a/5b[9:1]

In einem Dreihalskolben mit Rückflußkühler, Thermometer und Tropftrichter wurden 7 g (0.029 mol) Isolongifolenol (**4a/b**) (91 %ig nach GC) aus Beispiel 6 und 50 ml Toluol wasserfrei vorgelegt, über einen Zeitraum von 30 Min. portionsweise 1,2 g (0.03 mol) Natriumhydrid 60 %ig (in Paraffin) zugegeben, zum Sieden erhitzt und 1 Std. unter Rückfluß gerührt. Danach wurden innerhalb von 30 Min. 30,8 g (0.03 mol) Dimethylsulfat zugetropft, weitere 5 Std. unter Rückfluß gerührt, auf Raumtemperatur heruntergekühlt, mit je 50 g Wasser und 30 ml Ether versetzt, die organische Phase abgetrennt, die Wässer mit 30 ml Ether extrahiert, die organischen Phasen mit Wasser neutralgewaschen, über Na₂SO₄ getrocknet und das Lösungsmittel unter vermindertem Druck abdestilliert. Es verblieben 6,8 g Rohprodukt.

GC (Bedingungen siehe Beispiel 1): **5a** (78.1 %), **5b** (6.9 %)

### Beispiel 11

### Gewinnung von Isolongifolenylmethylether 5a

5 g des aus Beispiel 10 erhaltenen Rohgemisches wurden an einer Fischer-Spaltrohrkolonne destilliert. Ausbeute: 2,5 g.
D 20/4 = 0.9701
n 20/D = 1.4978
GC (Bedingungen siehe Beispiel 1): **5a** (98.4 %)

### GC/MS (Bedingungen siehe Beispiel 2)

**5a** Rₜ = 28.59 Min.
MS (70 eV), m/e (%): 234 (19, M⁺), 219 (20), 191 (10), 159 (11), 151 (100), 135 (21), 119 (10), 105 (12), 91 (14), 41 (12).
¹³C-NMR (CDCl₃) Varian VXR-300: δ [ppm]: 25.57, 26.26, 26.69, 28.41, 56.17 (CH₃), 24.71, 28.23, 36.97, 38.28 (CH₂), 46.56, 74.84, 110.91 (CH), 30.67, 42.26, 56.89, 159.99 (C).

### Beispiel 12

### Herstellung eines Gemisches von lsolongifolenylethylether 6a/b

In einem Dreihalskolben mit Tropftrichter, Thermometer und Rückflußkühler wurden 15 g (0.067 mol) Isolongifolenol (**4a/b**) (97.7 % nach GC) aus Beispiel 3 und 50 ml Toluol vorgelegt. Anschließend wurden unter Stickstoffatmosphäre über einen Zeitraum von 45 Min. portionsweise 3,2 g (0.08 mol) NaH 60 %ig (in Paraffin) zugegeben, zum Sieden erhitzt und 1 Std. unter Rückfluß gerührt. Danach wurden innerhalb von 1 Std. 10,4 g (0.08 mol) Diethylsulfat zugetropft, weitere 8 Std. unter Rückfluß gerührt, auf Raumtemperatur heruntergekühlt, mit Wasser versetzt und die organische Phase abgetrennt. Die Wässer wurden einmal mit 50 ml Ether extrahiert, die vereinigten organischen Phasen mit Wasser gewaschen, über Na₂SO₄ getrocknet und das Lösungsmittel unter vermindertem Druck abdestilliert. Es verblieben 16,3 g Rohprodukt.

### GC (Bedingungen siehe Beispiel 1): 6a (24.6 %), 6b (53.4 %)

Anschließende Feindestillation an einer Drehbandkolonne ergab 6,8 g Kp₂ₘₘ 141 - 143 °C.
D 20/4 = 0.9730
n 20/D = 1.4986

### GC (Bedingungen siehe Beispiel 1): 6a (30.1 %), 6b (68.1 %)

### GC/MS: Bedingungen siehe Beispiel 2

**6a** Rₜ = 27.66 Min.
MS (70 eV), m/e (%), 248 (28, M⁺), 233 (26), 205 (10), 165 (100), 149 (22), 137 (22), 119 (14), 105 (16), 91 (19), 41 (18).

**6b** Rₜ = 29.35 Min.
MS (70 eV), m/e (%), 248 (29, M+), 233 (27), 205 (10), 165 (100), 149 (22), 137 (23), 119 (15), 105 (17), 91 (20), 41 (19).

### Beispiel 13

### Herstellung eines Gemisches von Isolongifolenylpropylether 7a/7b

In einem Dreihalskolben mit Tropftrichter, Thermometer und Rückflußkühler wurden 15 g (0.067 mol) Isolongifolenol (**4a/b**) (97.70 % nach GC) aus Beispiel 3 und 50 ml Toluol vorgelegt, bei Raumtemperatur unter Stickstoffatmosphäre über einen Zeitraum von 45 Min. mit 3,2 g (0.08 mol) Natriumhydrid 60 %ig (in Paraffin) versetzt, zum Sieden erhitzt und 1 Std. unter Rückfluß gerührt. Anschließend wurde auf 70 °C heruntergekühlt, über einen Zeitraum von 15 Min. 7,90 g (0.08 mol) 1-Brompropan zugetropft, 4 Std. unter Rückfluß gerührt, auf Wasser ausgegossen, die organische Phase abgetrennt und die Wässer mit 50 ml Ether extrahiert. Die vereinigten organischen Phasen wurden einmal mit Wasser gewaschen, über Na₂SO₄ getrocknet und das Lösungsmittel unter vermindertem Druck abdestilliert. Es verblieben 14,8 g Rohprodukt.

### GC (Bedingungen siehe Beispiel 1): 7a (20.4 %), 7b (50.3 %)

Anschließende Feindestillation an einer Drehbandkolonne ergab 4,3 g Kp₂ₘₘ 144 - 146 °C.

### GC (Bedingungen siehe Beispiel 1): 7a (29.4 %), 7b (69.1 %)

### GC/MS (Bedingungen siehe Beispiel 2)

**7a** Rₜ = 30.26 Min.
MS (70 eV), m/e (%), 262 (52, M⁺), 247 (37), 179 (100), 163 (23), 147 (21), 137 (60), 121 (20), 119 (21), 105 (20), 91 (19), 41 (24).

**7b** Rt = 31.72 Min.
MS (70 eV), m/e (%), 262 (41, M⁺), 247 (31), 179 (100), 163 (23), 137 (65), 121 (22), 119 (19), 105 (21), 91 (25), 41 (36).

### Beispiel 14

### Herstellung eines Gemisches von Isolonqifolenylisopropylether 8a/b

Die Reaktion wurde analog der im Beispiel 12 aufgeführten Vorgehensweise, unter Zusatz von 2-Brompropan durchgeführt.

### GC/MS: Bedingungen siehe Beispiel 2

**8a** Rₜ = 27.93 Min.
MS (70 eV), m/e (%), 262 (32, M⁺), 220 (19), 205 (23), 177 (23), 163 (27), 137 (100), 121 (26), 107 (16), 91 (16), 41 (17).

**8b** Rt = 29.11 Min.
MS (70 eV), m/e (%), 262 (32, M⁺), 220 (18), 205 (22), 177 (22), 163 (27), 137 (100), 121 (26), 107 (16), 91 (15), 41 (17).

### Beispiel 15

### Herstellung eines Gemisches von Isolongifolenyl-n-butylether 9a/b

Die Reaktion wurde analog der im Beispiel 12 aufgeführten Vorgehensweise unter Zusatz von 1-Brombutan durchgeführt.

### GC/MS: Bedingungen siehe Beispiel 2

**9a** Rₜ = 32.19 Min.
MS (70 eV), m/e (%), 276 (45, M⁺), 261 (39), 203 (25), 193 (100), 177 (24), 137 (84), 121 (27), 105 (22), 91 (20), 41 (28).

**9b** Rₜ = 32.61 Min.
MS (70 eV), m/e (%), 276 (46, M⁺), 261 (39), 203 (25), 193 (100), 177 (24), 137 (84), 121 (27), 105 (22), 91 (22), 41 (28).

### Beispiel 16

### Herstellung eines Gemisches von lsolongifolenyl-sec-butylether 10a/b

Die Reaktion wurde analog der im Beispiel 12 aufgeführten Vorgehensweise unter Zusatz von 2-Brombutan durchgeführt.

### GC/MS: Bedingungen siehe Beispiel 2

**10a** Rₜ = 30.17 Min.
MS (70 eV), m/e (%), 276 (15, M⁺), 220 (21), 205 (17), 177 (24), 163 (21), 137 (100), 121 (21), 105 (13), 91 (14), 41 (15).

**10b** Rₜ = 30.55 Min.
MS (70 eV), m/e (%), 276 (15, M⁺), 220 (22), 205 (17), 177 (24), 163 (21), 137 (100), 121 (20), 105 (13), 91 (13), 41 (15).

### Beispiel 17

### Herstellung eines Gemisches von Isolongifolenylisobutylether 11a/b

Die Reaktion wurde analog der im Beispiel 12 aufgeführten Vorgehensweise unter Zusatz von 2-Brom-2-Methylpropan durchgeführt.

### GC/MS: Bedingungen siehe Beispiel 2

**11a** Rₜ = 27.96 Min.
MS (70 eV), m/e (%), 276 (6, M⁺), 220 (66), 203 (13), 177 (33), 163 (73), 137 (100), 121 (34), 105 (18), 91 (20), 41 (22).

**11b** Rₜ = 29.27 Min.
MS (70 eV), m/e (%), 276 (10, M⁺), 220 (72), 205 (16), 177 (40), 163 (80), 137 (100), 121 (39), 107 (18), 91 (16), 41 (16).

### Beispiel 18

### Herstellung eines Gemisches von Isolongufolenyl-tert-Butylether 12a/b

Die Reaktion wurde analog der in Beispiel 12 aufgeführten Vorgehensweise unter Zusatz von tert-Butylbromid durchgeführt.

### GC/MS: Bedingungen siehe Beispiel 2

**12a** Rₜ = 30.19 Min.
MS (70 eV), m/e (%), 276 (24, M⁺), 221 (60), 193 (67), 177 (50), 161 (49), 137 (100), 121 (50), 105 (51), 91 (57), 41 (34).

**12b** Rₜ = 31.38 Min.
MS (70 eV), m/e (%), 276 (16, M⁺), 221 (27), 205 (34), 193 (48), 177 (18), 137 (100), 121 (30), 105 (21), 91 (22), 41 (28).

### Beispiel 19

### Geruchsbeschreibung der Ether 5a/b bis 12a/b

Die Geruchsbewertung wurde von einem Expertengremium unter Verwendung von Riechstreifen vorgenommen.

**5a/b** aus Beispiel 8:
stark Moschus, pudrig, Cashmeran, Ambra, exaltierend, schwach holzig

**5a** aus Beispiel 11:
Moschus, leicht blumig, Cashmeran, exaltierend, würzig

**5b** aus Beispiel 9:
Ambra, trocken-Moschus, leicht würzig, schwach holzig-Patchouli

**6a/b** aus Beispiel 12:
trocken, leicht pudrig-fruchtig, feuchter Sand, Patchouli

**7a/b** aus Beispiel 13:
pudrig-Moschus, blumig, leicht erdig

**8 a/b** aus Beispiel 14:
säuerlich, pudrig-Moschus, trocken, schwach Patchouli-holzig

**9 a/b** aus Beispiel 15:
krautig, säuerlich, schwach grün-staubig

**10 a/b** aus Beispiel 16:
krautig, leicht säuerlich, staubig, trocken, schwach Moschus

**11 a/b** aus Beispiel 17:
schwach säuerlich-krautig, trocken , erdig

**12 a/b** aus Beispiel 18:
schwach pudrig-würzig, leicht metallisch-grün

### Beispiel 20

### Anwendungsbeispiel 1

### Parfümbase blumig-holzigen Typs

| | Gewichtsteile |
|---|---|
| Bergamottöl | 7,5 |
| Linalool | 4,0 |
| Phenylethylalkohol | 4,0 |
| Benzylacetat | 1,0 |
| Citronellol | 3,0 |
| Hedion ^{®} (a) | 10,0 |
| Lyral ^{®} (b) | 4,0 |
| Hydroxycitronellal | 3,0 |
| Rosenoxid L (c) | 2,5 |
| Hexylzimtaldehyd, alpha- | 7,5 |
| Ysamber-K^{®} (c) | 3,0 |
| Vetiverylacetat | 2,0 |
| Brahmanol-F (c) | 2,0 |
| Benzylsalicylat | 2,0 |
| Hexenylsalicylat, cis-3 | 1,0 |
| Cedramber (b) | 1,0 |
| Cindol 80 (c) | 0,5 |
| Oppoponaxextrakt | 0,5 |
| Eichenmoosextrakt 50 %ig in DPG | 7,0 |
| | 65,5 |

| | |
|---|---|
| (a) Firmenich (b) IFF (c) DRAGOCO | |

Die Parfümbase der angegebenen Formel besitzt insbesondere wegen der Anwesenheit von Ysamber K (einem zyklischen Isolongifolenyl-Ketal, wie es in der bereits erwähnten EP-A 0 543 470 offenbart ist) einen ausgewogenen blumigen-holzigen Duftcharakter, dessen Ausstrahlung in der Kopfnote durch Zugabe von 10 Teilen **5a/b** (aus Beispiel 8) deutlich und unter starker Betonung der blumigen Note verstärkt wird. Im Nachgeruch werden die weichen, pudrig samtigen Geruchseindrücke stärker betont, mit einem ausgeprägten Akzent in Richtung eines natürlichen Moschusduftes. Dabei ist insbesondere neben einer deutlichen Harmonisierung eine erhöhte Haftung des Nachgeruchs festzustellen.

### Beispiel 21

### Parfümbase vom Fougère-Typ

| | Gewichtsteile |
|---|---|
| Bergamottöl | 18,0 |
| Lavandinöl Super | 15,0 |
| Lilial ^{®} (b) | 10,0 |
| Anisaldehyd | 3,0 |
| Coumarin | 5,0 |
| Hexylzimtaldehyd, alpha- | 20,0 |
| Ambrinolepoxid, alpha- (c) | 0,5 |
| Ambrocenide 10^{®} (c) | 0,1 |
| Cantryl (c) | 5,0 |
| Pfefferminzöl | 1,0 |
| | 77,6 |

| | |
|---|---|
| (b) IFF (c) DRAGOCO | |

Die Parfümbase der angegebenen Formel besitzt einen frisch-krautigen Fougere-Duft. Die Zugabe von 10 Teilen 5a/b (aus Beispiel 8) verstärkt insbesondere die Kopfnote, wobei die Parfümbase deutlich an Intensität in Richtung blumig gewinnt. Die krautigen Aspekte verlieren an Intensität und erscheinen insgesamt abgerundeter und deutlich harmonisiert. Insbesondere im Nachgeruch ist ein deutlicher Akzent in Richtung eines natürlichen Moschusdufts festzustellen.

Die alternative Zugabe von 10 Teilen 6a/b bewirkt neben einer insgesamt abgerundeten Base eine deutliche Betonung langhaftender animalisch-erdiger Aspekte (Patchouli), wobei insbesondere die trocken-exaltierenden Geruchseindrücke eine Verstärkung erfahren.

## Patentansprüche

1. Cyclische Isolongifolenylether der allgemeinen Formel **A**, wobei geschlängelte Linien α- und β-Konfiguration sowie R folgende Reste bedeuten. 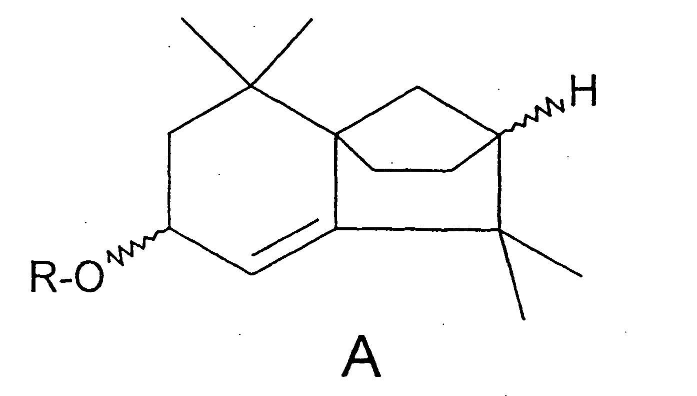 R= Me, Et, Pr, Iso-Pr, Bu, sec.-Bu, iso-Bu, tert-Bu

2. Cyclische Isolongifolenylether nach Anspruch 1, **dadurch gekennzeichnet, daß** der Wasserstoff am Brückenkopf der beiden Fünfringe α-konfiguriert ist.

3. Cyclische Isolongifolenylether nach Anspruch 1 oder 2, **dadurch gekennzeichnet, daß** R= Me, Et, Pr oder Iso-Pr, wobei die Konfiguration der Etherfunktion α oder β ist oder der cyclische Isolongifolenylether eine Mischung von Enantiomeren umfaßt, die sich in der Konfiguration der Etherfunktion unterscheiden.

4. Cyclische Isolongifolenylether nach einem der vorangehenden Ansprüche , **dadurch gekennzeichnet, daß** R = Me, wobei die Konfiguration der Methyletherfunktion α oder β ist oder der cyclische lsolongifolenyl-Methylether eine Mischung von Enantiomeren umfaßt, die sich in der Konfiguration der Methyletherfunktion unterscheiden.

5. Verfahren zur Herstellung eines cyclischen Isolongifolenylethers nach einem der vorangehenden Ansprüche, **dadurch gekennzeichnet, dass** Isolongifolenol in enantiomerenreiner Form oder als Enantiomerengemisch entweder unter säurekatalysierten Bedingungen mit einem aliphatischen Alkohol (Alkylhydroxid) oder unter basenkatalysierten Bedingungen mit einem Alkylhalogenid oder Alkylsulfat in einem aprotischen Lösungsmittel umgesetzt wird, wobei die jeweilige Alkylfunktion dem Rest R des cyclischen Isolongifolenylethers entspricht.

6. Verwendung eines cyclischen Isolongifolenylethers nach einem der Ansprüche 1-4 als Riechstoff oder Bestandteil von Riechstoffmischungen bzw. Parfümölen zur Parfümierung von kosmetischen oder technischen Gebrauchsgütern.

7. Verwendung eines cyclischen Isolongifolenylethers nach einem der Ansprüche 1-4 als Mittel zur Verstärkung der Kopfnote einer Parfümkomposition.

8. Verwendung eines cyclischen lsolongifolenylethers nach einem der Ansprüche 1-4 als Mittel zur Abrundung und/oder Harmonisierung der Hauptnoten einer Riechstoffkomposition.

9. Verwendung eines cyclischen Isolongifolenylethers nach Anspruch 1 mit R= Me, Et, Pr oder Iso-Pr als Mittel zum Vermitteln oder Verstärken eines Moschus- und/oder Patchouliaspekts in einer Riechstoffkomposition.

## Claims

1. Cyclic isolongifolenyl ethers of the general formula A, wherein wavy lines signify α and β configuration, and R signifies the following radicals: 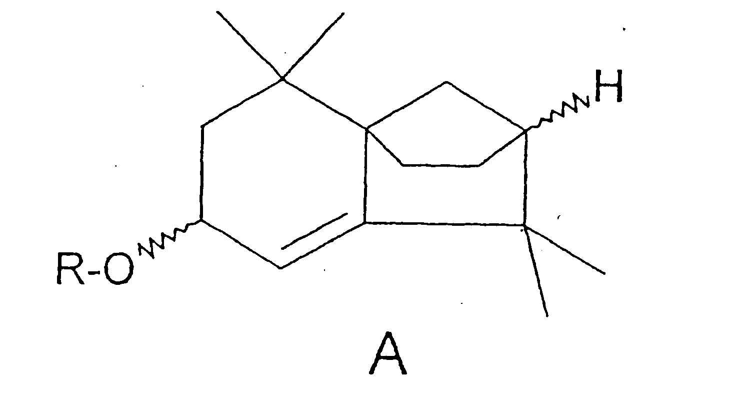 R = Me, Et, Pr, iso-Pr, Bu, sec-Bu, iso-Bu, tert-Bu.

2. Cyclic isolongifolenyl ethers according to claim 1, **characterised in that** the hydrogen at the bridgehead of the two five-membered rings is α-configured.

3. Cyclic isolongifolenyl ethers according to claim 1 or 2, **characterised in that** R = Me, Et, Pr or iso-Pr, wherein the configuration of the ether function is α or β or the cyclic isolongifolenyl ether comprises a mixture of enantiomers which differ in the configuration of the ether function.

4. Cyclic isolongifolenyl ethers according to one of the preceding claims, **characterised in that** R = Me, wherein the configuration of the methyl ether function is α or β or the cyclic isolongifolenyl methyl ether comprises a mixture of enantiomers which differ in the configuration of the methyl ether function.

5. A process for the preparation of a cyclic isolongifolenyl ether according to one of the preceding claims, **characterised in that** isolongifolenol is reacted in an enantiomerically pure form or as a mixture of enantiomers, either under acid-catalysed conditions with an aliphatic alcohol (alkyl hydroxide) or under base-catalysed conditions with an alkyl halide or alkyl sulfate in an aprotic solvent, each alkyl function corresponding to the radical R of the cyclic isolongifolenyl ether.

6. The use of a cyclic isolongifolenyl ether according to one of claims 1 - 4 as a fragrance or component of fragrance mixtures or perfume oils for perfuming cosmetic or technical consumer goods.

7. The use of a cyclic isolongifolenyl ether according to one of claims 1 - 4 as a means of intensifying the top note of a perfume composition.

8. The use of a cyclic isolongifolenyl ether according to one of claims 1 - 4 as a means of rounding off and/or harmonising the main notes of a fragrance composition.

9. The use of a cyclic isolongifolenyl ether according to claim 1 with R = Me, Et, Pr or iso-Pr as a means of conveying or intensifying a musk and/or patchouli aspect in a fragrance composition.

## Revendications

1. Ethers isolongifolényliques cycliques de formule générale A, dans laquelle les lignes sinueuses indiquent les configurations α et β et R représente les groupes ci-après : 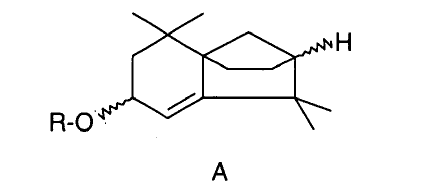 R = méthyle, éthyle, propyle, isopropyle, butyle, sec-butyle, isobutyle, tert-butyle.

2. Ethers isolongifolényliques cycliques selon la revendication 1, **caractérisés en ce que** l'hydrogène sur la tête de pont des deux cycles pentagonaux est en configuration α.

3. Ethers isolongifolényliques cycliques selon la revendication 1 ou 2, **caractérisés en ce que** R représente un groupe méthyle, éthyle, propyle ou isopropyle, la fonction éther est en configuration α ou β, ou bien l'éther isolongifolénylique cyclique consiste en un mélange d'énantiomères qui diffèrent par la configuration de la fonction éther.

4. Ethers isolongifolényliques cycliques selon l'une des revendications qui précèdent, **caractérisés en ce que** R représente un groupe méthyle, la fonction éther méthylique étant en configuration α ou β ou bien l'éther isolongifolényl-méthylique cyclique consiste en un mélange d'énantiomères dont la configuration de la fonction éther méthylique est différente.

5. Procédé pour la préparation d'un éther isolongifolénylique cyclique selon l'une des revendications qui précèdent, **caractérisé en ce que** l'on fait réagir l'isolongifolénol à l'état d'énantiomère pur ou à l'état de mélange d'énantiomères, soit avec catalyse acide, avec un alcool aliphatique (hydroxyde d'alkyle) soit, avec catalyse basique, avec un halogénure d'alkyle ou un sulfate d'alkyle, dans un solvant aprotonique, la fonction alkyle du réactif correspondant au groupe R de l'éther isolongifolénylique cyclique.

6. Utilisation d'un éther isolongifolénylique cyclique selon l'une des revendications 1 à 4 en tant que matière odorante ou constituant de mélanges de matières odorantes ou de parfums pour le parfumage de produits de consommation cosmétiques ou techniques.

7. Utilisation d'un éther isolongifolénylique cyclique selon l'une des revendications 1 à 4 en tant que composant servant à renforcer la note de tête d'une composition de parfum.

8. Utilisation d'un éther isolongifolénylique cyclique selon l'une des revendications 1 à 4 en tant que composant pour arrondir et/ou harmoniser la note de tête d'une composition de parfum.

9. Utilisation d'un éther isolongifolénylique cyclique selon la revendication 1 pour lequel R = méthyle, éthyle, propyle ou isopropyle, en tant que composant pour conférer ou renforcer un aspect musc et/ou patchouli dans une composition de parfum.
